(19) Europäisches Patentamt
European Patent Office
Office européen des brevets

(11) **EP 2 609 871 A1**

(12) **EUROPEAN PATENT APPLICATION**

(43) Date of publication:
**03.07.2013 Bulletin 2013/27**

(51) Int Cl.:
*A61B 8/06* (2006.01)    *A61B 8/08* (2006.01)
*A61B 6/00* (2006.01)    *G01S 15/89* (2006.01)

(21) Application number: **12199553.4**

(22) Date of filing: **27.12.2012**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **28.12.2011 KR 20110144432**

(71) Applicant: **Samsung Medison Co., Ltd.
Gangwon-do 250-870 (KR)**

(72) Inventor: **Choi, Seok Won
135-851 Seoul (KR)**

(74) Representative: **Lorenz, Markus
Lorenz & Kollegen
Patentanwälte Partnerschaftsgesellschaft
Alte Ulmer Strasse 2
89522 Heidenheim (DE)**

(54) **Providing vector doppler image based on decision data in ultrasound system**

(57)    There are provided embodiments for providing a vector Doppler image based on decision data. In one embodiment, by way of non-limiting example, an ultrasound system comprises: a processing unit configured to form vector information and additional information of a target objet based on ultrasound data corresponding to the target object, wherein set decision data corresponding to the target object based on the additional information, the processing unit being further configured to form a vector Doppler mode image based on the decision data and the vector information.

## FIG. 15

**Description**

CROSS-REFERENCE TO RELATED APPLICATIONS

The present application claims priority from Korean Patent Application No. 10-2011-0144432 filed on December 28, 2011.

TECHNICAL FIELD

The present disclosure generally relates to ultrasound systems, and more particularly to providing a vector Doppler image based on decision data in an ultrasound system.

BACKGROUND

An ultrasound system has become an important and popular diagnostic tool since it has a wide range of applications. Specifically, due to its non-invasive and non-destructive nature, the ultrasound system has been extensively used in the medical profession. Modem high-performance ultrasound systems and techniques are commonly used to produce two-dimensional or three-dimensional ultrasound images of internal features of target objects (e.g., human organs).

The ultrasound system provides ultrasound images of various modes including a brightness mode image representing reflection coefficients of ultrasound signals (i.e., ultrasound echo signals) reflected from a target object of a living body with a two-dimensional image, a Doppler mode image representing velocity of a moving target object with spectral Doppler by using a Doppler effect, a color Doppler mode image representing velocity of the moving target object with colors by using the Doppler effect, an elastic image representing mechanical characteristics of tissues before and The ultrasound system transmits the ultrasound signals to the living body and receives the ultrasound echo signals from the living body to form Doppler signals corresponding to a region of interest, which is set on the brightness mode image. The ultrasound system further forms the color Doppler mode image representing the velocity of the moving target object with colors based on the Doppler signals. In particular, the color Doppler image represents the motion of the target object (e.g., blood flow) with the colors. The color Doppler image is used to diagnose disease of a blood vessel, a heart and the like. However, it is difficult to represent an accurate motion of the target object (e.g., blood flow) since the respective colors indicated by a motion value is a function of the velocity of the target object, which moves forward in a transmission direction of the ultrasound signals and moves backward in the transmission direction of the ultrasound signals.

To resolve this problem, a vector Doppler method capable of obtaining the velocity and direction of the blood flow is used. A cross beam-based method of the vector Doppler method acquires velocity magnitude components from at least two different directions, and combine the velocity magnitude components to detect vector information having a two-dimensional or three-dimensional direction information and a magnitude information.

SUMMARY

There are provided embodiments for providing a vector Doppler image based on the decision data.

In one embodiment, by way of non-limiting example, an ultrasound system comprises: a processing unit configured to form vector information and additional information of a target objet based on ultrasound data corresponding to the target object, wherein set decision data corresponding to the target object based on the additional information, the processing unit being further configured to form a vector Doppler mode image based on the decision data and the vector information.

In another embodiment, there is provided a method of providing a vector Doppler image, comprising: a) forming vector information and additional information of a target object based on ultrasound data corresponding to the target object; b) setting decision data corresponding to target object based on the additional information; and c) forming a vector Doppler mode image based on the decision data and the vector information.

The Summary is provided to introduce a selection of concepts in a simplified form that are further described below in the Detailed Description. This Summary is not intended to identify key or essential features of the claimed subject matter, nor is it intended to be used in determining the scope of the claimed subject matter.

BRIEF DESCRIPTION OF THE DRAWINGS

FIG. 1 is a block diagram showing an illustrative embodiment of an ultrasound system.

FIG. 2 is a schematic diagram showing an example of a brightness mode image and a region of interest.

FIG. 3 is a block diagram showing an illustrative embodiment of an ultrasound data acquiring unit.

FIGS. 4 to 7 are schematic diagrams showing examples of transmission directions and reception directions.

FIG. 8 is a schematic diagram showing an example of sampling data and pixels of an ultrasound image.

FIGS. 9 to 12 are schematic diagrams showing examples of performing a reception beam-forming.

FIG. 13 is a schematic diagram showing an example of setting weights.

FIG. 14 is a schematic diagram showing an example of setting a sampling data set.

FIG. 15 is a flow chart showing a process of forming a vector Doppler image based on decision data.

FIG. 16 is a schematic diagram showing an example of the transmission directions, the reception directions, the vector information and an over-determined problem.

## DETAILED DESCRIPTION

A detailed description may be provided with reference to the accompanying drawings. One of ordinary skill in the art may realize that the following description is illustrative only and is not in any way limiting.

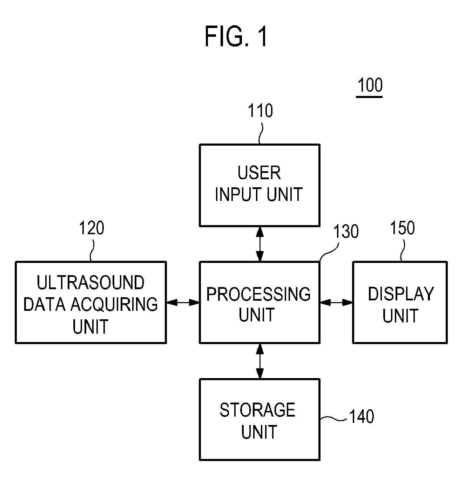

Referring to FIG. 1, an ultrasound system 100 in accordance with an illustrative embodiment is shown. As depicted therein, the ultrasound system 100 includes a user input unit 110.

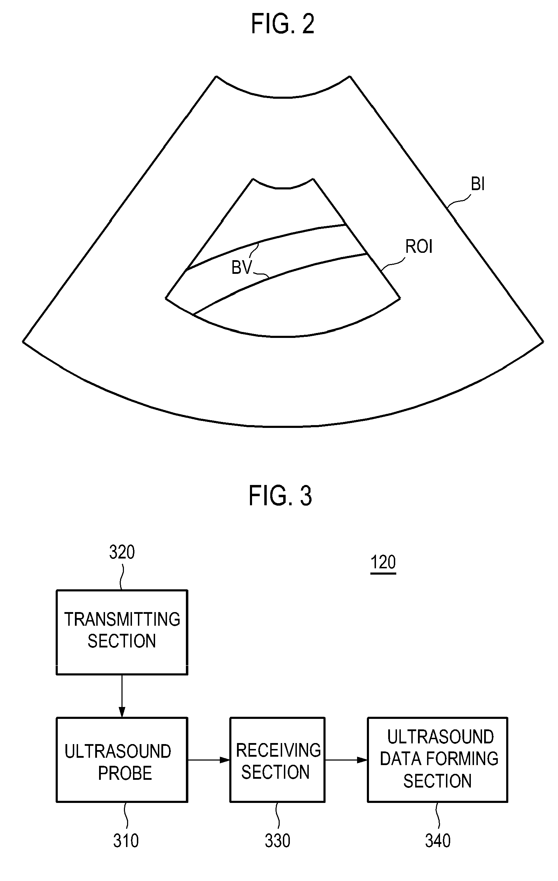

The user input unit 110 is configured to receive input information from a user. In one embodiment, the input information includes information for setting a region of interest ROI on a brightness mode image BI, as shown in FIG. 2. However, it should be noted herein that the input information may not be limited thereto. The region of interest ROI includes a color box for obtaining a two-dimensional or three-dimensional vector Doppler image. In FIG. 2, the reference numeral BV represents a blood vessel. The user input unit 110 includes a control panel, a track ball, a touch screen, a mouse, a keyboard and the like.

The ultrasound system 100 further includes an ultrasound data acquiring unit 120. The ultrasound data acquiring unit 120 is configured to transmit ultrasound signals to a living body. The living body includes target objects (e.g., blood vessel, heart, blood flow, etc). The ultrasound data acquiring unit 120 is further configured to receive ultrasound signals (i.e., ultrasound echo signals) from the living body to acquire ultrasound data corresponding to an ultrasound image.

FIG. 3 is a block diagram showing an illustrative embodiment of the ultrasound data acquiring unit. Referring to FIG. 3, the ultrasound data acquiring unit 120 includes an ultrasound probe 310.

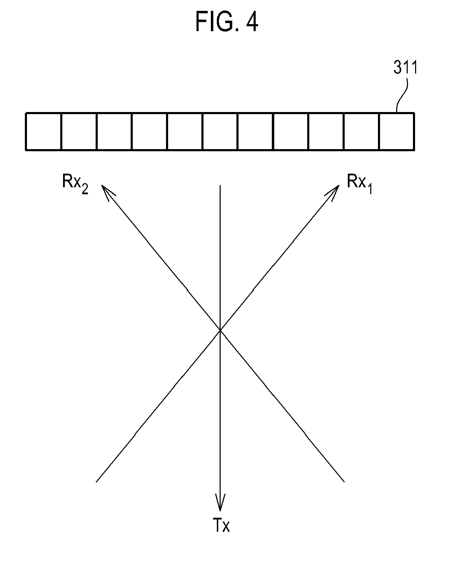

The ultrasound probe 310 includes a plurality of elements 311 (see FIG. 4) for reciprocally converting between ultrasound signals and electrical signals. The ultrasound probe 310 is configured to transmit the ultrasound signals to the living body. The ultrasound signals transmitted from the ultrasound probe 310 are plane wave signals that the ultrasound signals are not focused at a focusing point, or focused signals that the ultrasound signals are focused at the focusing point. However, it should be noted herein that the ultrasound signals may not be limited thereto. The ultrasound probe 310 is further configured to receive the ultrasound echo signals from the living body to output electrical signals (hereinafter referred to as "reception signals"). The reception signals are analog signals. The ultrasound probe 310 includes a convex probe, a linear probe and the like.

The ultrasound data acquiring unit 120 further includes a transmitting section 320. The transmitting section 320 is configured to control the transmission of the ultrasound signals. The transmitting section 320 is further configured to generate electrical signals (hereinafter referred to as "transmission signals") for obtaining the ultrasound image in consideration of the elements 311.

In one embodiment, the transmitting section 320 is configured to generate transmission signals (hereinafter referred to as "brightness mode transmission signals") for obtaining the brightness mode image BI in consideration of the elements 311. Thus, the ultrasound probe 310 is configured to convert the brightness mode transmission signals provided from the transmitting section 320 into the ultrasound signals, transmit the ultrasound signals to the living body, and receive the ultrasound echo signals from the living body to output reception signals (hereinafter referred to as "brightness mode reception signals").

The transmitting section 320 is further configured to generate transmission signals (hereinafter referred to as "Doppler mode transmission signals") corresponding to an ensemble number in consideration of the elements 311 and at least one transmission direction of the ultrasound signals (i.e., transmission beam). Thus, the ultrasound probe 310 is configured to convert the Doppler mode transmission signals provided from the transmitting section 320 into the ultrasound signals, transmit the ultrasound signals to the living body in the at least one transmission signals, and receive the ultrasound echo signals from the living body to output reception signals (hereinafter referred to as "Doppler mode reception signals"). The ensemble number represents the number of transmitting and receiving the ultrasound signals.

As one example, the transmitting section 320 is configured to generate the Doppler mode transmission signals corresponding to the ensemble number in consideration of a transmission direction Tx and the elements 311, as shown in FIG. 4. The transmission direction is one of a direction (i.e., 0 degree) perpendicular to a longitudinal direction of the elements 311 to a maximum steering direction of the transmission beam.

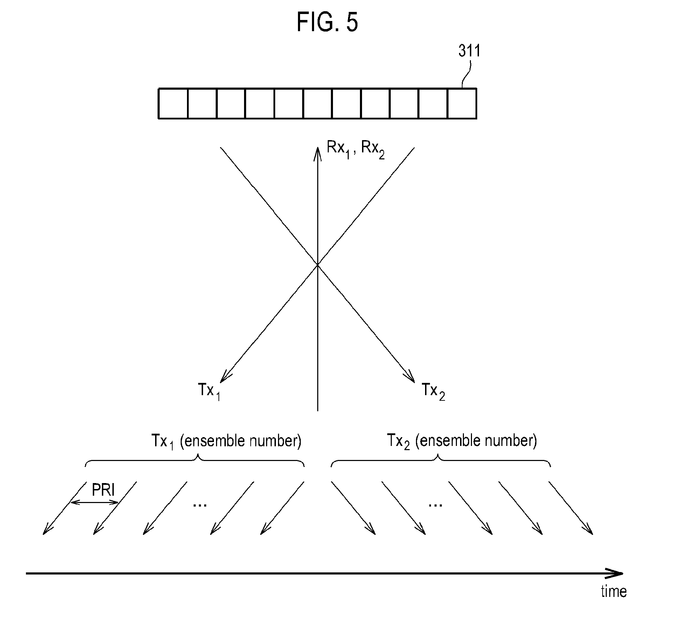

As another example, the transmitting section 320 is configured to generate first Doppler mode transmission signals corresponding to the ensemble number in consideration of a first transmission direction $Tx_1$ and the elements 311, as shown in FIG. 5. Thus, the ultrasound probe 310 is configured to convert the first Doppler mode transmission signals provided from the transmitting section 320 into the ultrasound signals, transmit the ultrasound signals to the living body in the first transmission direction $Tx_1$, and receive the ultrasound echo signals from the living body to output first Doppler mode reception signals. The transmitting section 320 is further configured to generate second Doppler mode transmission signals corresponding to the ensemble number in consideration of a second transmission direction $Tx_2$ and the elements 311, as shown in FIG. 5. Thus, the ultrasound probe 310 is configured to convert the second Doppler mode transmission signals provided from the transmitting section 320 into the ultrasound signals, transmit the ultrasound signals to the living body in the second transmission direction $Tx_2$, and receive the ultrasound echo signals from the living body to output second Doppler mode reception signals. In FIG. 5, the reference numeral RPI represents a pulse repeat interval.

In another embodiment, the transmitting section 320 is configured to generate the brightness mode transmission signals for obtaining the brightness mode image BI in consideration of the elements 311. Thus, the ultrasound probe 310 is configured to convert the brightness mode transmission signals provided from the transmitting section 320 into the ultrasound signals, transmit the ultrasound signals to the living body, and receive the ultrasound echo signals from the living body to output the brightness mode reception signals.

The transmitting section 320 is further configured to generate the Doppler mode transmission signals corresponding to the ensemble number in consideration of the at least one transmission direction and the elements 311. Thus, the ultrasound probe 310 is configured to convert the Doppler mode transmission signals provided from the transmitting section 320 into the ultrasound signals, transmit the ultrasound signals to the living body, and receive the ultrasound echo signals from the living body to output the Doppler mode reception signals. The ultrasound signals are transmitted in an interleaved transmission scheme. The interleaved transmission scheme will be described below in detail.

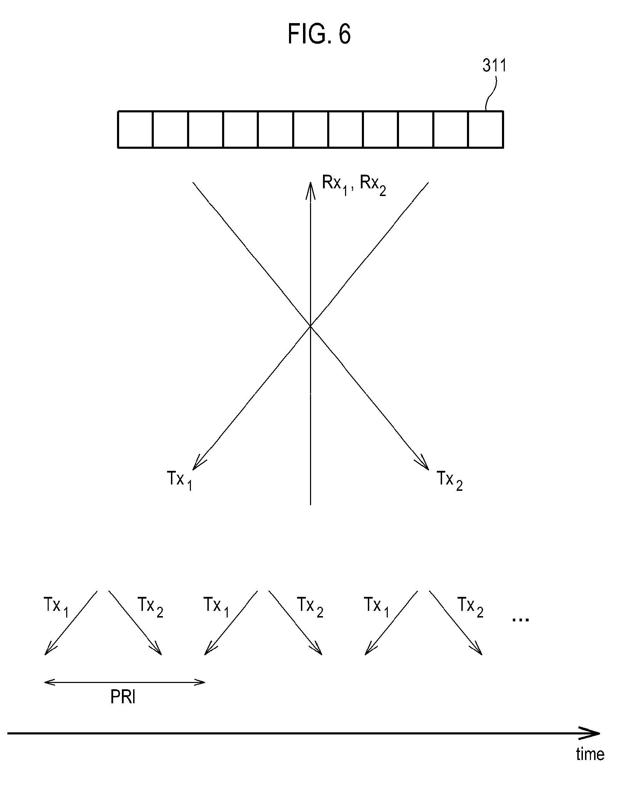

For example, the transmitting section 320 is configured to generate the first Doppler mode transmission signals in consideration of the first transmission direction $Tx_1$ and the elements 311, as shown in FIG. 6. Thus, the ultrasound probe 310 is configured to convert the first Doppler mode transmission signals provided from the transmitting section 320 into the ultrasound signals, and transmit the ultrasound signals to the living body in the first transmission direction $Tx_1$. Then, the transmitting section 320 is further configured to generate the second Doppler mode transmission signals in consideration of the second transmission direction $Tx_2$ and the elements 311, as shown in FIG. 6. Thus, the ultrasound probe 310 is configured to convert the second Doppler mode transmission signals provided from the transmitting section 320 into the ultrasound signals, and transmit the ultrasound signals to the living body in the second transmission direction $Tx_2$. The ultrasound probe 310 is further configured to receive the ultrasound echo signals (i.e., ultrasound echo signals corresponding to first Doppler mode transmission signals) from the living body to output the first Doppler mode reception signals. The ultrasound probe 310 is further configured to receive the ultrasound echo signals (i.e., ultrasound echo signals corresponding to second Doppler mode transmission signals) from the living body to output the second Doppler mode reception signals.

Thereafter, the transmitting section 320 is configured to generate the first Doppler mode transmission signals based on the pulse repeat interval, as shown in FIG. 6. Thus, the ultrasound probe 310 is configured to convert the first Doppler mode transmission signals provided from the transmission section 320 into the ultrasound signals, and transmit the ultrasound signals to the living body in the first transmission direction $Tx_1$. Then, the transmitting section 320 is further configured to generate the second Doppler mode transmission signals based on the pulse repeat interval, as shown in FIG. 6. Accordingly, the ultrasound probe 310 is configured to convert the second Doppler mode transmission signals provided from the transmitting section 320 into the ultrasound signals, and transmit the ultrasound signals to the living body in the second transmission direction $Tx_2$. The ultrasound probe 310 is further configured to receive the ultrasound echo signals (i.e., ultrasound echo signals corresponding to first Doppler mode transmission signals) from the living body to output the first Doppler mode reception signals. The ultrasound probe 310 is further configured to receive the ultrasound echo signals (i.e., ultrasound echo signals corresponding to second Doppler mode transmission signals) from the living body to output the second Doppler mode reception signals.

As described above, the transmitting section 320 is configured to generate the first Doppler mode transmission signals and the second Doppler mode transmission signals corresponding to the ensemble number.

In yet another embodiment, the transmitting section 320 is configured to generate the brightness mode transmission signals for obtaining the brightness mode image BI in consideration of the elements 311. Thus, the ultrasound probe 310 is configured to convert the brightness mode transmission signals provided from the transmitting section 320 into the ultrasound signals, transmit the ultrasound signals to the living body, and receive the ultrasound echo signals from the living body to output the brightness mode reception signals.

The transmitting section 320 is further configured to generate the Doppler mode transmission signals corresponding to the ensemble number in consideration of the at least one transmission direction and the elements 311. Thus, the ultrasound probe 310 is configured to convert the Doppler mode transmission signals provided from the transmitting section 320 into the ultrasound signals, transmit the ultrasound signals to the living body in the at least one transmission direction, and receive the ultrasound echo signals from the living body to output the Doppler mode reception signals. The ultrasound signals are transmitted according to the pulse repeat interval.

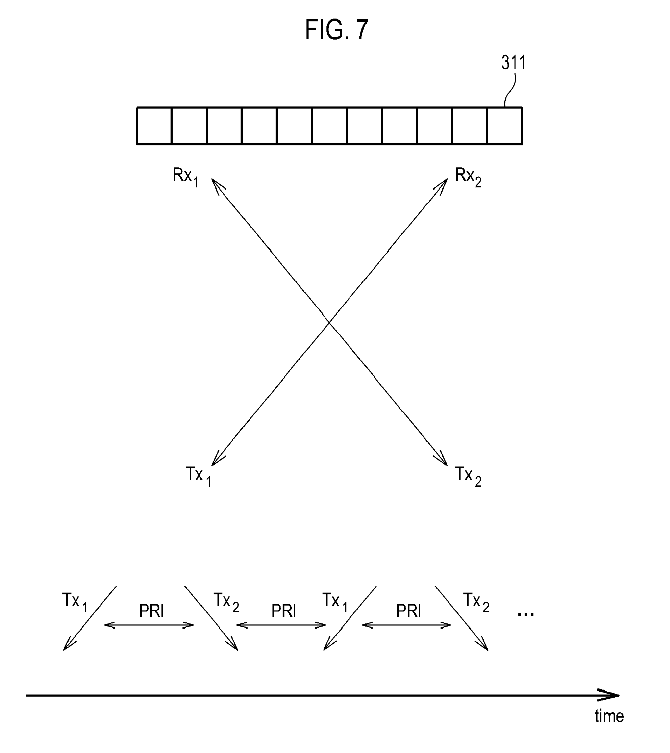

For example, the transmitting section 320 is configured to generate the first Doppler mode transmission signals in consideration of the first transmission direction $Tx_1$ and the elements 311 based on the pulse repeat interval, as shown in FIG. 7. Thus, the ultrasound probe 310 is configured to convert the first Doppler mode transmission signals provided from the transmitting section 320 into the ultrasound signals, transmit the ultrasound signals to the living body in the first transmission direction $Tx_1$, and receive the ultrasound echo signals from the living body to output the first Doppler mode reception signals. The transmitting section 320 is further configured to generate the second Doppler mode transmission signals in consideration of the second transmission direction $Tx_2$ and the elements 311 based on the pulse repeat interval, as shown in FIG. 7. Thus, the ultrasound probe 310 is configured to convert the second Doppler mode transmission signals provided from the transmitting section 320 into the ultrasound signals, transmit the ultrasound signals to the living body in the second transmission direction $Tx_2$, and receive the ultrasound echo signals from the living body to output the second Doppler mode reception signals.

As described above, the transmitting section 320 is configured to generate the first Doppler mode transmission signals and the second Doppler mode transmission signals corresponding to the ensemble number based on the pulse repeat interval.

Referring back to FIG. 3, the ultrasound data acquiring unit 120 further includes a receiving section 330. The receiving section 330 is configured to perform an analog-digital conversion upon the reception signals provided from the ultrasound probe 310 to form sampling data. The receiving section 330 is further configured to perform a reception beam-forming upon the sampling data in consideration of the elements 311 to form reception-focused data. The reception beam-forming will be described below in detail.

In one embodiment, the receiving section 330 is configured to perform the analog-digital conversion upon the brightness mode reception signals provided from the ultrasound probe 310 to form sampling data (hereinafter referred to as "brightness mode sampling data"). The receiving section 330 is further configured to perform the reception beam-forming upon the brightness mode sampling data to form reception-focused data (hereinafter referred to as "brightness mode reception-focused data").

The receiving section 330 is further configured to perform the analog-digital conversion upon the Doppler mode reception signals provided from the ultrasound probe 310 to form sampling data (hereinafter referred to as "Doppler mode sampling data"). The receiving section 330 is further configured to perform the reception beam-forming upon the Doppler mode sampling data to form reception-focused data (hereinafter referred to as "Doppler mode reception-focused data") corresponding to at least one reception direction of the ultrasound echo signals (i.e., reception beam).

As one example, the receiving section 330 is configured to perform the analog-digital conversion upon the Doppler mode reception signals provided from the ultrasound probe 310 to form the Doppler mode sampling data. The receiving section 330 is further configured to perform the reception beam-forming upon the Doppler mode sampling data to form first Doppler mode reception-focused data corresponding to a first reception direction $Rx_1$ and second Doppler mode reception-focused data corresponding to a second reception direction $Rx_2$, as shown in FIG. 4.

As another example, the receiving section 330 is configured to perform the analog-digital conversion upon the first Doppler mode reception signals provided from the ultrasound probe 310 to form first Doppler mode sampling data corresponding to the first transmission direction $Tx_1$, as shown in FIG. 5. The receiving section 330 is further configured to perform the reception beam-forming upon the first Doppler mode sampling data to form the first Doppler mode reception-focused data corresponding to the first reception direction $Rx_1$. The receiving section 330 is also configured to perform the analog-digital conversion upon the second Doppler mode reception signals provided from the ultrasound probe 310 to form second Doppler mode sampling data corresponding to the second transmission direction $Tx_2$, as shown in FIG. 5. The receiving section 330 is further configured to perform the reception beam-forming upon the second Doppler mode sampling data to form the second Doppler mode reception-focused data corresponding to the second reception direction $Rx_2$. If the reception direction is perpendicular to the elements 311 of the ultrasound probe 310, then a maximum aperture size is used.

The reception beam-forming is described with reference to the accompanying drawings.

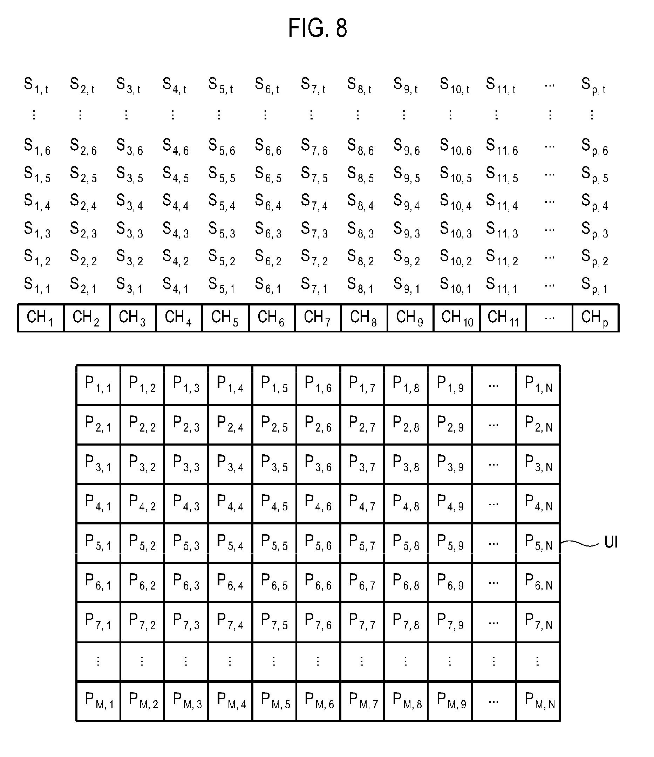

In one embodiment, the receiving section 330 is configured to perform the analog-digital conversion upon the reception signals provided through a plurality of channels $CH_k$, wherein $1 \leq k \leq N$, from the ultrasound probe 310 to form sampling data $S_{i,j}$, wherein the i and j are a positive integer, as shown in FIG.8. The sampling data $S_{i,j}$ are stored in a storage unit 140. The receiving section 330 is further configured to detect pixels corresponding to the sampling data based on positions of the elements 311 and positions (orientation) of pixels of the ultrasound image UI with respect to the elements 311. That is, the receiving section 330 selects the pixels, which the respective sampling data are used as pixel data thereof, during the reception beam-forming based on the positions of the elements 311 and the orientation of the respective pixels of the ultrasound image UI with respect to the elements 311. The receiving section 330 is configured to cumulatively assign the sampling data corresponding to the selected pixels as the pixel data.

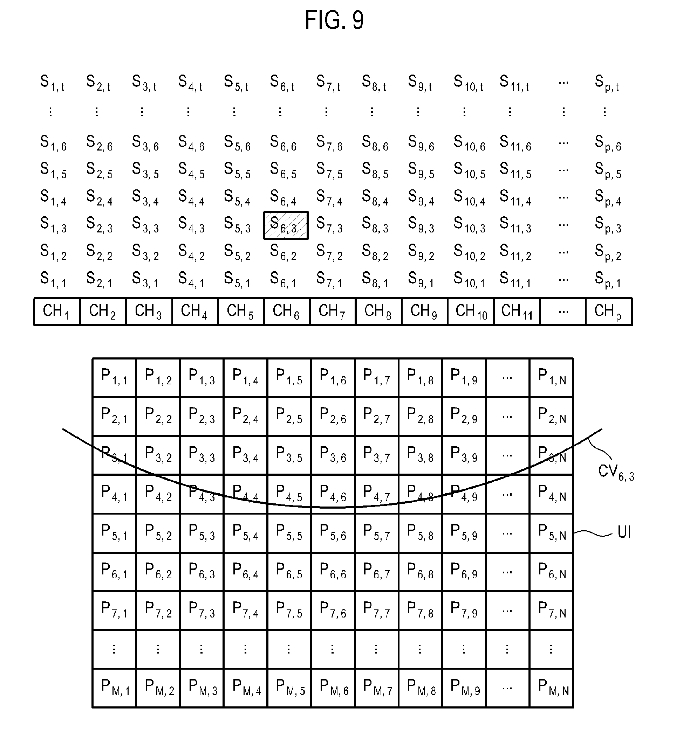

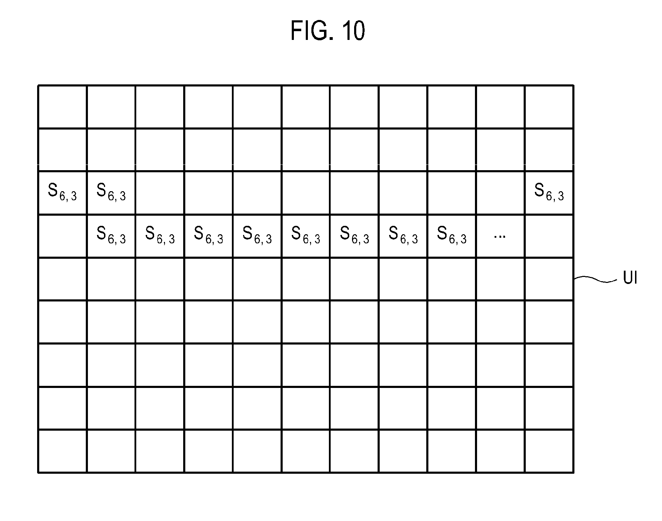

For example, the receiving section 330 is configured to set a curve (hereinafter referred to as "reception beam-forming curve") $CV_{6,3}$ for selecting pixels, which the sampling data $S_{6,3}$ are used as the pixel data thereof, during the reception beam-forming based on the positions of the elements 311 and the orientation of the respective pixels of the ultrasound image UI with respect to the elements 311, as shown in FIG. 9. The receiving section 330 is further configured to detect the pixels $P_{3,1}$, $P_{3,2}$, $P_{4,2}$, $P_{4,3}$, $P_{4,4}$, $P_{4,5}$, $P_{4,6}$, $P_{4,7}$, $P_{4,8}$, $P_{4,9}$, ... $P_{3,N}$ corresponding to the reception beam-forming curve $CV_{6,3}$ from the pixels $P_{a,b}$ of the ultrasound image UI, wherein $1 \leq a \leq M$, $1 \leq b \leq N$. That is, the receiving section 330 selects the pixels $P_{3,1}$, $P_{3,2}$, $P_{4,2}$, $P_{4,3}$, $P_{4,4}$, $P_{4,5}$, $P_{4,6}$, $P_{4,7}$, $P_{4,8}$, $P_{4,9}$, ... $P_{3,N}$ on which the reception beam-forming curve $CV_{6,3}$ passes among the pixels $P_{a,b}$ of the ultrasound image UI. The receiving section 330 is also configured to assign the sampling data $S_{6,3}$ to the selected pixels $P_{3,1}$, $P_{3,2}$, $P_{4,2}$, $P_{4,3}$, $P_{4,4}$, $P_{4,5}$, $P_{4,6}$, $P_{4,7}$, $P_{4,8}$, $P_{4,9}$, ... $P_{3,N}$, as shown in FIG. 10.

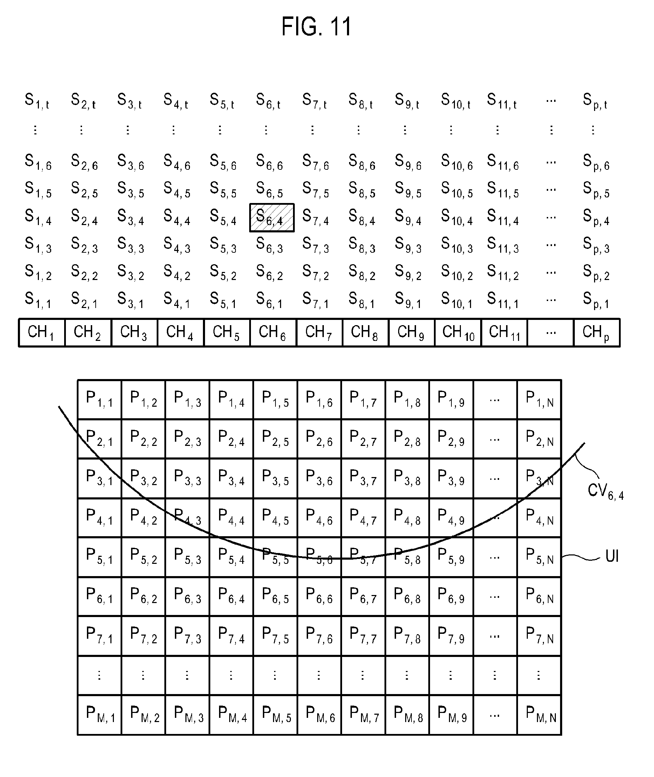

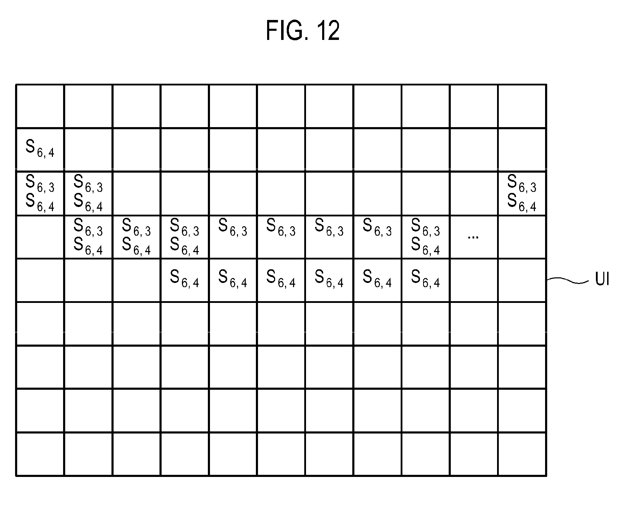

Thereafter, the receiving section 330 is configured to set a reception beam-forming curve $CV_{6,4}$ for selecting pixels, which the sampling data $S_{6,4}$ are used as the pixel data thereof, during the reception beam-forming based on the positions of the elements 311 and the orientation of the respective pixels of the ultrasound image UI with respect to the elements 311, as shown in FIG. 11. The receiving section 330 is further configured to detect the pixels $P_{2,1}$, $P_{3,1}$, $P_{3,2}$, $P_{4,2}$, $P_{4,3}$, $P_{4,4}$, $P_{5,4}$, $P_{5,5}$, $P_{5,6}$, $P_{5,7}$, $P_{5,8}$, $P_{4,9}$, $P_{5,9}$, ... $P_{4,N}$, $P_{3,N}$ corresponding to the reception beam-forming curve $CV_{6,4}$ from the pixels $P_{a,b}$ of the ultrasound image UI. That is, the receiving section 330 selects the pixels $P_{2,1}$, $P_{3,1}$, $P_{3,2}$, $P_{4,2}$, $P_{4,3}$, $P_{4,4}$, $P_{5,4}$, $P_{5,5}$, $P_{5,6}$, $P_{5,7}$, $P_{5,8}$, $P_{4,9}$, $P_{5,9}$, ... $P_{4,N}$, $P_{3,N}$ on which the reception beam-forming curve $CV_{6,4}$ passes among the pixels $P_{a,b}$ of the ultrasound image UI. The receiving section 330 is further configured to assign the sampling data $S_{6,4}$ to the selected pixels $P_{2,1}$, $P_{3,1}$, $P_{3,2}$, $P_{4,2}$, $P_{4,3}$, $P_{4,4}$, $P_{5,4}$, $P_{5,5}$, $P_{5,6}$, $P_{5,7}$, $P_{5,8}$, $P_{5,9}$, ... $P_{4,N}$, $P_{3,N}$, as shown in FIG. 12. In this way, the respective sampling data, which are used as the pixel data, are cumulatively assigned to the pixels as the pixel data.

The receiving section 330 is configured to perform the reception beam-forming (i.e., summing) upon the sampling data, which are cumulatively assigned to the respective pixels $P_{a,b}$ of the ultrasound image UI to form the reception-focused data.

In another embodiment, the receiving section 330 is configured to perform the analog-digital conversion upon the reception signals provided through the plurality of channels $CH_k$ from the ultrasound probe 310 to form the sampling data $S_{i,j}$, as shown in FIG. 8. The sampling data $S_{i,j}$ are stored in the storage unit 140. The receiving section 330 is further configured to detect pixels corresponding to the sampling data based on the positions of the elements 311 and the position (orientation) of the pixels of the ultrasound image UI with respect to the elements 311. That is, the receiving section 330 selects the pixels, which the respective sampling data are used as the pixel data thereof, during the reception beam-forming based on the positions of the elements 311 and the orientation of the respective pixels of the ultrasound image UI with respect to the elements 311. The receiving section 330 is configured to cumulatively assign the sampling data corresponding to the selected pixels as the pixel data. The receiving section 330 is further configured to determine pixels existing in the same column among the selected pixels. The receiving section 330 is also configured to set weights corresponding to the respective determined pixels. The receiving section 330 is additionally configured to apply the weights to the sampling data of the respective pixels.

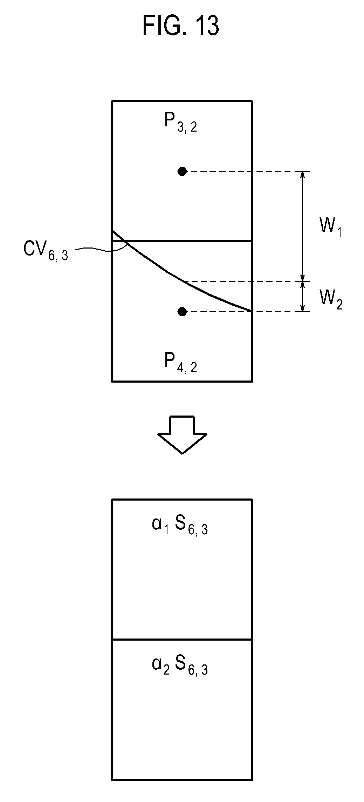

For example, the receiving section 330 is configured to set the reception beam-forming curve $CV_{6,3}$ for selecting pixels, which the sampling data $S_{6,3}$ are used as the pixel data thereof, during the reception beam-forming based on the positions of the elements 311 and the orientation of the respective pixels of the ultrasound image UI with respect to the elements 311, as shown in FIG. 9. The receiving section 330 is further configured to detect the pixels $P_{3,1}$, $P_{3,2}$, $P_{4,2}$, $P_{4,3}$, $P_{4,4}$, $P_{4,5}$, $P_{4,6}$, $P_{4,7}$, $P_{4,8}$, $P_{4,9}$, ... $P_{3,N}$ corresponding to the reception beam-forming curve $CV_{6,3}$ from the pixels $P_{a,b}$ of the ultrasound image UI, wherein $1 \leq a \leq M$, $1 \leq b \leq N$. That is, the receiving section 330 selects the pixels $P_{3,1}$, $P_{3,2}$, $P_{4,2}$, $P_{4,3}$, $P_{4,4}$, $P_{4,5}$, $P_{4,6}$, $P_{4,7}$, $P_{4,8}$, $P_{4,9}$, ... $P_{3,N}$ on which the reception beam-forming curve $CV_{6,3}$ passes among the pixels $P_{a,b}$ of the ultrasound image UI. The receiving section 330 is also configured to assign the sampling data $S_{6,3}$ to the selected pixels $P_{3,1}$, $P_{3,2}$, $P_{4,2}$, $P_{4,3}$, $P_{4,4}$, $P_{4,5}$, $P_{4,6}$, $P_{4,7}$, $P_{4,8}$, $P_{4,9}$, ... $P_{3,N}$, as shown in FIG. 10. The receiving section 330 is further configured to determine pixels $P_{3,2}$ and $P_{4,2}$, which exist in the same column among the selected pixels $P_{3,1}$, $P_{3,2}$, $P_{4,2}$, $P_{4,3}$, $P_{4,4}$, $P_{4,5}$, $P_{4,6}$, $P_{4,7}$, $P_{4,8}$, $P_{4,9}$, ... $P_{3,N}$. The receiving section 330 is further configured to calculate a distance $W_1$ from a center of the determined pixel $P_{3,2}$ to the reception beam-forming curve $CV_{6,3}$ and a distance $W_2$ from a center of the determined pixel $P_{4,2}$ to the reception beam-forming curve $CV_{6,3}$, as shown in FIG. 13. The receiving section 330 is additionally configured to set a first weight $\alpha_1$ corresponding to the pixel $P_{3,2}$ based on the distance $W_1$ and a second weight $\alpha_2$ corresponding to the pixel $P_{4,2}$ based on the distance $W_2$. The first weight $\alpha_1$ and the second weight $\alpha_2$ are set to be in proportional to or in inverse proportional to the calculated distances. The receiving section 330 is further configured to apply the first weight $\alpha_1$ to the sampling data $S_{6,3}$ assigned to the pixel $P_{3,2}$ and to apply the second weight $\alpha_2$ to the sampling data $S_{6,3}$ assigned to the pixel $P_{4,2}$. The receiving section 330 is configured to perform the above process upon the remaining sampling data.

The receiving section 330 is configured to perform the reception beam-forming upon the sampling data, which are cumulatively assigned to the respective pixels $P_{a,b}$ of the ultrasound image UI to form the reception-focused data.

In yet another embodiment, the receiving section 330 is configured to perform the analog-digital conversion upon the reception signals provided through the plurality of channels $CH_k$ from the ultrasound probe 310 to form the sampling data $S_{i,j}$, as shown in FIG. 8. The sampling data $S_{i,j}$ are stored in the storage unit 140. The receiving section 330 is further configured to set a sampling data set based on the sampling data $S_{i,j}$. That is, The receiving section 330 sets the sampling data set for selecting pixels, which the sampling data $S_{i,j}$ are used as the pixel data thereof, during the reception beam-forming.

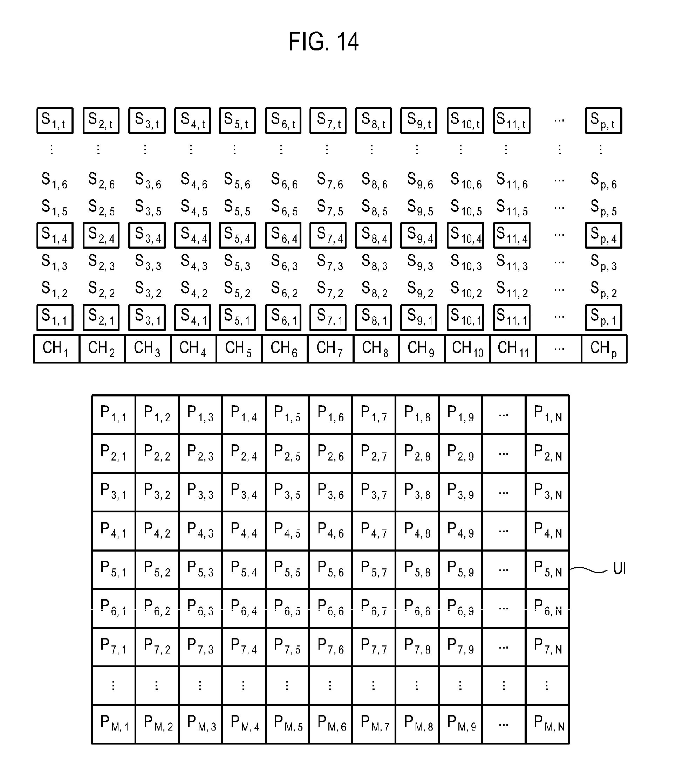

For example, the receiving section 330 is configured to set the sampling data $S_{1,1}$, $S_{1,4}$, ... $S_{1,t}$, $S_{2,1}$, $S_{2,4}$, ... $S_{2,t}$, ... $S_{p,t}$ as the sampling data set (denoted by a box) for selecting the pixels, which the sampling data $S_{i,j}$ are used as the pixel data thereof, during the reception beam-forming, as shown in FIG. 14.

The receiving section 330 is further configured to detect the pixels corresponding to the respective sampling data of the sampling data set based on the positions of the elements 311 and the positions (orientation) of the respective pixels of the ultrasound image UI with respect to the elements 311. That is, the receiving section 330 selects the pixels, which the respective sampling data of the sampling data set are used as the pixel data thereof, during the reception beam-forming based on the positions of the elements 311 and the orientation of the respective pixels of the ultrasound image UI with respect to the elements 311. The receiving section 330 is further configured to cumulatively assign the sampling data to the selected pixels in the same manner with the above embodiments. The receiving section 330 is also configured to perform the reception beam-forming upon the sampling data, which are cumulatively assigned to the respective pixels of the ultrasound image UI to form the reception-focused data.

In yet another embodiment, the receiving section 330 is configured to perform a down-sampling upon the reception signals provided through the plurality of channels $CH_k$ from the ultrasound probe 310 to form down-sampling data. As described above, the receiving section 330 is further configured to detect the pixels corresponding to the respective sampling data, based on the positions of the elements 311 and the positions (orientation) of the respective pixels of the ultrasound image UI with respect to the elements 311. That is, the receiving section 330 selects the pixels, which the respective sampling data are used as the pixel data thereof, during the reception beam-forming based on the positions of the elements 311 and the orientation of the pixels of the ultrasound image UI with respect to the elements 311. The receiving section 330 is further configured to cumulatively assign the respective sampling data to the selected pixels in the same manner of the above embodiments. The receiving section 330 is further configured to perform the reception beam-forming upon the sampling data, which are cumulatively assigned to the respective pixels of the ultrasound image UI to form the reception-focused data.

However, it should be noted herein that the reception beam-forming may not be limited thereto.

Referring back to FIG. 3, the ultrasound data acquiring unit 120 further includes an ultrasound data forming section 340. The ultrasound data forming section 340 is configured to form the ultrasound data corresponding to the ultrasound image based on the reception-focused data provided from the receiving section 330. The ultrasound data forming section 340 is further configured to perform a signal process (e.g., gain control, etc) upon the reception-focused data.

In one embodiment, the ultrasound data forming section 340 is configured to form ultrasound data (hereinafter referred to as "brightness mode ultrasound data") corresponding to the brightness mode image based on the brightness mode reception-focused data provided from the receiving section 330. The brightness mode ultrasound data include radio frequency data.

The ultrasound data forming section 340 is further configured to form ultrasound data (hereinafter referred to as "Doppler mode ultrasound data") corresponding to the region of interest ROI based on the Doppler mode reception-focused data provided from the receiving section 330. The Doppler mode ultrasound data include in-phase/quadrature data. However, it should be noted herein that the Doppler mode ultrasound data may not be limited thereto.

For example, the ultrasound data forming section 340 forms first Doppler mode ultrasound data based on the first Doppler mode reception-focused data provided from the receiving section 330. The ultrasound data forming section 340 further forms second Doppler mode ultrasound data based on the second Doppler mode reception-focused data provided from the receiving section 330.

Referring back to FIG. 1, the ultrasound system 100 further includes a processing unit 130 in communication with the user input unit 110 and the ultrasound data acquiring unit 120. The processing unit 130 includes a central processing unit, a microprocessor, a graphic processing unit and the like.

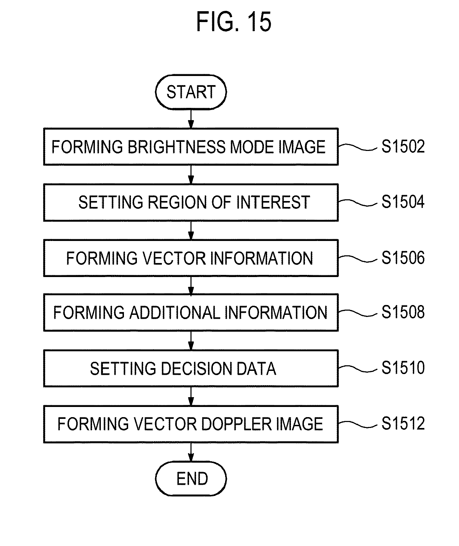

FIG. 15 is a flow chart showing a process of forming a vector Doppler image based on decision data. The processing unit 130 is configured to form the brightness mode image BI based on the brightness mode ultrasound data provided from the ultrasound data acquiring unit 120, at step S1502 in FIG. 15. The brightness mode image BI is displayed on a display unit 150.

The processing unit 130 is configured to set the region of interest ROI on the brightness mode image BI based on the input information provided from the user input unit 110, at step S1504 in FIG. 15. Thus, the ultrasound data acquiring unit 120 is configured to transmit the ultrasound signals to the living body and receive the ultrasound echo signals from the living body to acquire the Doppler mode ultrasound data, in consideration of the region of interest ROI.

The processing unit 130 is configured to form vector information based on the Doppler mode ultrasound data provided from the ultrasound data acquiring unit 120, at step S1506 in FIG. 15. That is, the processing unit 130 forms the vector information corresponding to motion (i.e., velocity and direction) of the target object based on the Doppler mode ultrasound data.

Generally, when the transmission direction of the ultrasound signals is equal to the reception direction of the ultrasound echo signals and a Doppler angle is θ, the following relationship is established:

$$X \cos \theta = \frac{C_0 f_d}{2 f_0} \qquad (1)$$

In equation 1, X represents a reflector velocity (i.e., velocity of target object), $C_0$ represents a sound speed in the living body, $f_d$ represents a Doppler shift frequency, and fo represents an ultrasound frequency.

The Doppler shift frequency $f_d$ is calculated by the difference between a frequency of the ultrasound signals (i.e., transmission beam) and a frequency of the ultrasound echo signals (i.e., reception beam). Also, the velocity component Xcosθ projected to the transmission direction is calculated by the equation 1.

When the transmission direction of the ultrasound signals (i.e., transmission beam) is different to the reception direction of the ultrasound echo signals (i.e., reception beam), the following relationship is established:

$$X \cos \theta_T + X \cos \theta_R = \frac{C_0 f_d}{f_0} \qquad (2)$$

In equation 2, $\theta_T$ represents an angle between the ultrasound signals (i.e., transmission beam) and the blood flow, and $\theta_R$ represents an angle between the ultrasound echo signals (i.e., reception beam) and the blood flow.

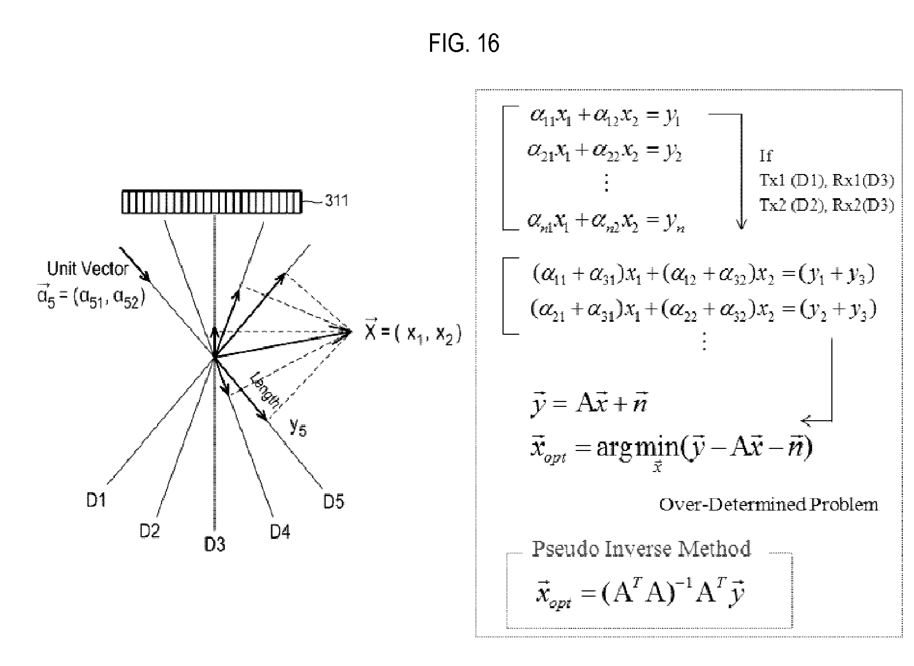

FIG. 16 is a schematic diagram showing an example of the transmission directions, the reception directions, the vector information and an over-determined problem. Referring to FIG. 16, when the ultrasound signals (i.e., transmission beam) are transmitted in a first direction D1 and the ultrasound echo signals (i.e., reception beam) are received in the first direction D1, the following relationship is established:

$$\overrightarrow{\alpha_1} \overline{X} = \alpha_{11} x_1 + \alpha_{12} x_2 = y_1 = X \cos \theta \qquad (3)$$

In equation 3, $\overrightarrow{\alpha_1} = (\alpha_{11}, \alpha_{12})$ represents a unit vector of the first direction D1, $\overline{X} = (x_1, x_2)$ represents variables, and $y_1$ is calculated by equation 1.

When the ultrasound signals (i.e., transmission beam) are transmitted in a second direction D2 and the ultrasound echo signals (i.e., reception beam) are received in a third direction D3, the following relationship is established:

$$(\alpha_{21} + \alpha_{31}) x_1 + (\alpha_{22} + \alpha_{32}) x_2 = (y_2 + y_3) = X \cos \theta_2 + X \cos \theta_3 \qquad (4)$$

Equations 3 and 4 assume a two-dimensional environment. However, equations 3 and 4 are expanded to a three-dimensional environment. That is, when expanding equations 3 and 4 to the three-dimensional environment, the following relationship is established:

$$\alpha_{11} x_1 + \alpha_{12} x_2 + \alpha_{13} x_3 = y \qquad (5)$$

In the case of the two-dimensional environment (i.e., two-dimensional vector), at least two equations are required to calculate the variables $x_1$ and $x_2$. For example, when the ultrasound signals (i.e., transmission beam) are transmitted in the third direction D3 and the ultrasound echo signals (i.e., reception beam) are received in the second direction D2 and a fourth direction D4 as shown in FIG. 16, the following equations are established:

$$(\alpha_{31} + \alpha_{21})x_1 + (\alpha_{32} + \alpha_{22})x_2 = (y_3 + y_2)$$

$$(\alpha_{31} + \alpha_{41})x_1 + (\alpha_{32} + \alpha_{42})x_2 = (y_3 + y_4) \quad\quad (6)$$

The vector $\vec{X} = (x_1, x_2)$ is calculated by the equations of equation 6.

When the reception beam-forming is performed in at least two angles (i.e., at least two reception directions), at least two equations are obtained and represented as the over-determined problem, as shown in FIG. 16. The over-determined problem is well known in the art. Thus, it has not been described in detail so as not to unnecessarily obscure the present disclosure. The over-determined problem is solved by a pseudo inverse method, a weighted least square method and the like based on noise characteristics added to the Doppler shift frequency. That is, M×N equations are obtained by M transmission directions and the reception beam-forming of N reception directions at every transmission.

The processing unit 130 is configured to form additional information corresponding to the target object based on the Doppler mode ultrasound data provided from the ultrasound data acquiring unit 120, at step S1508 in FIG. 15. In one embodiment, the additional information includes at least one of power information (or variance information), intensity value (i.e., brightness value) information and Doppler information of the target object.

Optionally, the processing unit 130 is configured to form the vector information and the additional information simultaneously based on the ultrasound data (i.e., Doppler mode ultrasound data).

Further optionally, the processing unit 130 is configured to form the additional information based on the ultrasound data (i.e., Doppler mode ultrasound data). The processing unit 130 is further configured to the vector information based on the ultrasound data (i.e., Doppler mode ultrasound data).

The processing unit 130 is configured to set the decision data based on the additional information, at step S1510 in FIG. 15. The decision data are data for determining whether the target object exists or not, and for determining the amount of the target object. That is, the decision data are the data for performing a filtering process upon the vector information.

In one embodiment, the processing unit 130 is configured to detect an intensity value (i.e., brightness value) corresponding to the target object (e.g., blood vessel BV) based on the additional information. The processing unit 130 is further configured to set the detected brightness value as the decision data for performing the filtering process upon the vector information. That is, the processing unit 130 sets the detected brightness value as the decision data for filtering (i.e., transparent-process) the blood vessel BV from the vector Doppler image.

In another embodiment, the processing unit 130 is configured to set a power threshold value based on the additional information. The methods of setting the power threshold value are well known in the art. Thus, they have not been described in detail so as not to unnecessarily obscure the present disclosure. The processing unit 130 is further configured to set the decision data for performing the filtering process upon the vector information based on the power threshold value. That is, the processing unit 130 sets the decision data for filtering (i.e., removing) vector information corresponding to power, which is less than or equal to the power threshold value.

In yet another embodiment, the processing unit 130 is configured to set the power threshold value based on the additional information. The processing unit 130 is further configured to set the decision data for performing the filtering process upon the vector information based on the power threshold value. That is, the processing unit 130 sets the decision data for filtering (i.e., transparent process) vector information corresponding to power, which is less than or equal to the power threshold value.

In yet another embodiment, the processing unit 130 is configured to set the power threshold value based on the additional information. The processing unit 130 is further configured to set the decision data for performing the filtering process upon the vector information based on the power threshold value. The filtering process includes an alpha blending process. However, it should be noted herein that the filtering process may not be limited thereto. That is, the processing unit 130 sets the decision data for undermining vector information corresponding to power, which is less than or equal to the power threshold, and for reinforcing vector information corresponding to power, which is more than the power threshold value.

In yet another embodiment, the processing unit 130 is configured to set the power threshold value based on the additional information. The processing unit 130 is further configured to set the decision data for performing the filtering process upon the vector information based on the power threshold value. That is, the processing unit 130 sets the decision data for reinforcing vector information corresponding to power, which is less than or equal to the power threshold value, and for undermining vector information corresponding to power, which is more than the power threshold value.

However, it should be noted herein that the methods of setting the decision data may not be limited thereto.

The processing unit 130 is configured to form the vector Doppler image based on the decision data and the vector information, at step S1512 in FIG. 15. The vector Doppler image includes a vector Doppler image for representing the vector information as a color wheel, a vector Doppler image for representing a magnitude of the vector information as a length and representing a direction of the vector information as an arrow, a vector Doppler image for representing the motion of the target object as motion of a particle and the like.

In one embodiment, the processing unit 130 is configured to perform the filtering process for filtering the target object (e.g., blood vessel BV) based on the decision data upon the vector information. The processing unit 130 is further configured to form the vector Doppler image based on the filtering-processed vector information.

In another embodiment, the processing unit 130 is configured to form a decision data curve for performing the filtering process upon vector information corresponding to the power, which is less than or equal to the power threshold value, based on the decision data. The processing unit 130 is further configured to perform the filtering process upon the vector information based on the decision data curve. That is, the processing unit 130 applies the decision data curve to the vector information. The processing unit 130 is further configured to form the vector Doppler image based on the filtering-processed vector information.

In yet another embodiment, the processing unit 130 is configured to detect vector information corresponding to the power, which is less than or equal to the power threshold value, based on the decision data. The processing unit 130 is further configured to perform the filtering process (i.e., transparent process) upon the detected vector information. The processing unit 130 is further configured to form the vector Doppler image based on the filtering-processed vector information.

In yet another embodiment, the processing unit 130 is configured to form the decision data curve for undermining vector information corresponding to the power, which is less than or equal to the power threshold value, and for reinforcing vector information corresponding to the power, which is more than the power threshold value.

As one example, the processing unit 130 forms the decision data curve for adjusting at least one of a particle density, a particle size and a particle tail according to the amount of the target object (e.g., blood flow, etc) based on the decision data.

As another example, the processing unit 130 forms the decision data curve for adjusting at least one of an arrow size, an arrow density, an arrow length and a color according to the amount of the target object based on the decision data.

As yet another example, the processing unit 130 forms the decision data curve for adjusting at least one of a streamline density, a streamline color and a streamline alpha-blending according to the amount of the target object based on the decision data.

As yet another example, the processing unit 130 forms the decision data curve for adjusting at least one of a thickness, a density, an alpha-blending and a viscosity of a profile line according to the amount of the target object based on the decision data.

The processing unit 130 is further configured to perform the filtering process upon the vector information based on the decision data curve. That is, the processing unit 130 applies the decision data curve to the vector information. The processing unit 130 is further configured to form the vector Doppler image based on the filtering-processed vector information.

In yet another embodiment, the processing unit 130 is configured to form the decision data curve for reinforcing vector information corresponding to the power, which is less than or equal to the power threshold value, and for undermining vector information corresponding to the power, which is more than the power threshold value, based on the decision data. The processing unit 130 is further configured to perform the filtering process upon the vector information based on the decision data curve. That is, the processing unit 130 applies the decision data curve to the vector information. The processing unit 130 is further configured to form the vector Doppler image based on the filtering-processed vector information.

Referring back to FIG. 1, the ultrasound system 100 further includes the storage unit 140. The storage unit 140 stores the ultrasound data (i.e., brightness mode ultrasound data and Doppler mode ultrasound data) acquired by the ultrasound data acquiring unit 120. The storage unit 140 further stores the vector information formed by the processing unit 130.

The ultrasound system 100 further includes the display unit 150. The display unit 150 is configured to display the brightness mode image BI formed by the processing unit 130. The display unit 150 is further configured to display the vector Doppler image formed by the processing unit 130.

Although embodiments have been described with reference to a number of illustrative embodiments thereof, it should be understood that numerous other modifications and embodiments can be devised by those skilled in the art that will fall within the scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

**Claims**

1. An ultrasound system, comprising:

   a processing unit configured to form vector information and additional information of a target objet based on ultrasound data corresponding to the target object, wherein set decision data corresponding to the target object based on the additional information, the processing unit being further configured to form a vector Doppler mode image based on the decision data and the vector information.

2. The ultrasound system of Claim 1, wherein the processing unit is configured to form the vector information corresponding to a velocity and a direction of the target object in consideration of at least one transmission direction and at least one reception direction corresponding to the at least one transmission direction based on the ultrasound data.

3. The ultrasound system of Claim 1, wherein the additional information includes at least one of power information, variance information, intensity value information and Doppler information of the target object.

4. The ultrasound system of Claim 3, wherein the processing unit is configured to:

   detect the intensity value information corresponding to the target object based on the ultrasound data;
   set the decision data based on the intensity value information;
   perform a filtering process for filtering vector information corresponding to the target object upon the vector information based on the decision data; and
   form the vector Doppler image based on the filtering-processed vector information.

5. The ultrasound system of Claim 3, wherein the processing unit is configured to:

   set a power threshold value based on the additional information;
   set the decision data for filtering vector information corresponding to power, which is less than or equal to the power threshold value;
   form a decision data curve for filtering vector information corresponding to the power, which is less than or equal to the power threshold value, based on the decision data;
   apply the decision data curve to the vector information; and
   form the vector Doppler image based on the filtering-processed vector information.

6. The ultrasound system of Claim 3, wherein the processing unit is configured to:

   set a power threshold value based on the additional information;
   set the decision data for performing a transparent process upon vector information corresponding to power, which is less than or equal to the power threshold value;
   perform the transparent process upon vector information corresponding to power, which is less than or equal to the power threshold value, based on the decision data; and
   form the vector Doppler image based on the vector information.

7. The ultrasound system of Claim 3, wherein the processing unit is configured to:

   set a power threshold value based on the additional information; and
   set the decision data for reinforcing vector information corresponding to power, which is more than the power threshold value;
   form a decision data curve for performing a filtering process based on the decision data;
   apply the decision data curve to the vector information; and
   form the vector Doppler image based on the filtering-processed vector information.

8. The ultrasound system of Claim 3, wherein the processing unit is configured to:

set a power threshold value based on the additional information;
set the decision data for reinforcing vector information corresponding to power, which is less than or equal to the power threshold value;
form a decision data curve for performing a filtering process based on the decision data;
apply the decision data curve to the vector information; and
form the vector Doppler image based on the filtering-processed vector information.

9. A method of providing a vector Doppler image, comprising:

   a) forming vector information and additional information of a target object based on ultrasound data corresponding to the target object;
   b) setting decision data corresponding to target object based on the additional information; and
   c) forming a vector Doppler mode image based on the decision data and the vector information.

10. The method of Claim 9, wherein the step a) comprises:

    forming the vector information corresponding to a velocity and a direction of the target object in consideration of at least one transmission direction and at least one reception direction corresponding to the at least one transmission direction based on the ultrasound data.

11. The method of Claim 9, wherein the additional information includes at least one of power information, variance information, intensity value information and Doppler information of the target object.

12. The method of Claim 11, wherein the step b) comprises:

    detecting the intensity value information corresponding to the target object based on the ultrasound data; and
    setting the decision data based on the intensity value information, and
    wherein the step c) comprises:

       performing a filtering process for filtering vector information corresponding to the target object upon the vector information based on the decision data; and
       forming the vector Doppler image based on the filtering-processed vector information.

13. The method of Claim 11, wherein the step b) comprises:

    setting a power threshold value based on the additional information; and
    setting the decision data for filtering vector information corresponding to power, which is less than or equal to the power threshold value, and
    wherein the step c) comprises:

       forming a decision data curve for filtering vector information corresponding to power, which is less than or equal to the power threshold value, based on the decision data;
       applying the decision data curve to the vector information;
       forming the vector Doppler image based on the filtering-processed vector information.

14. The method of Claim 11, wherein the step b) comprises:

    setting a power threshold value based on the additional information; and
    setting the decision data for performing a transparent process upon vector information corresponding to power, which is less than or equal to the power threshold value, and
    wherein the step c) comprises:

       performing the transparent process upon vector information corresponding to power, which is less than or equal to the power threshold value, based on the decision data; and
       forming the vector Doppler image based on the vector information.

15. The method of Claim 11, wherein the step b) comprises:

setting a power threshold value based on the additional information; and
setting the decision data for reinforcing vector information corresponding to power, which is more than the power threshold value, and
wherein the step c) comprises:

forming a decision data curve for performing a filtering process based on the decision data;
applying the decision data curve to the vector information; and
forming the vector Doppler image based on the filtering-processed vector information.

**16.** The method of Claim 11, wherein the step b) comprises:

setting a power threshold value based on the additional information; and
setting the decision data for reinforcing vector information corresponding to power, which is less than or equal to the power threshold value, and
wherein the step c) comprises:

forming a decision data curve performing a filtering process based on the decision data;
applying the decision data curve to the vector information; and
forming the vector Doppler image based on the filtering-processed vector information.

# FIG. 1

100

110

USER
INPUT UNIT

120

ULTRASOUND
DATA ACQUIRING
UNIT

130

150

PROCESSING
UNIT

DISPLAY
UNIT

STORAGE
UNIT

140

# FIG. 2

BI

ROI

BV

# FIG. 3

320

120

| TRANSMITTING SECTION |
|:--:|

| ULTRASOUND PROBE | → | RECEIVING SECTION | → | ULTRASOUND DATA FORMING SECTION |
|:--:|:--:|:--:|:--:|:--:|

310

330

340

FIG. 4

FIG. 5

311

Rx$_1$, Rx$_2$

Tx$_1$

Tx$_2$

Tx$_1$ (ensemble number)

Tx$_2$ (ensemble number)

PRI

...

...

time

# FIG. 6

# FIG. 7

311

Rx₁        Rx₂

Tx₁        Tx₂

Tx₁   PRI   Tx₂   PRI   Tx₁   PRI   Tx₂   ...

time

# FIG. 8

| $S_{1,t}$ | $S_{2,t}$ | $S_{3,t}$ | $S_{4,t}$ | $S_{5,t}$ | $S_{6,t}$ | $S_{7,t}$ | $S_{8,t}$ | $S_{9,t}$ | $S_{10,t}$ | $S_{11,t}$ | $\cdots$ | $S_{p,t}$ |
|---|---|---|---|---|---|---|---|---|---|---|---|---|
| $\vdots$ | $\vdots$ | $\vdots$ | $\vdots$ | $\vdots$ | $\vdots$ | $\vdots$ | $\vdots$ | $\vdots$ | $\vdots$ | $\vdots$ | $\vdots$ | $\vdots$ |
| $S_{1,6}$ | $S_{2,6}$ | $S_{3,6}$ | $S_{4,6}$ | $S_{5,6}$ | $S_{6,6}$ | $S_{7,6}$ | $S_{8,6}$ | $S_{9,6}$ | $S_{10,6}$ | $S_{11,6}$ | $\cdots$ | $S_{p,6}$ |
| $S_{1,5}$ | $S_{2,5}$ | $S_{3,5}$ | $S_{4,5}$ | $S_{5,5}$ | $S_{6,5}$ | $S_{7,5}$ | $S_{8,5}$ | $S_{9,5}$ | $S_{10,5}$ | $S_{11,5}$ | $\cdots$ | $S_{p,5}$ |
| $S_{1,4}$ | $S_{2,4}$ | $S_{3,4}$ | $S_{4,4}$ | $S_{5,4}$ | $S_{6,4}$ | $S_{7,4}$ | $S_{8,4}$ | $S_{9,4}$ | $S_{10,4}$ | $S_{11,4}$ | $\cdots$ | $S_{p,4}$ |
| $S_{1,3}$ | $S_{2,3}$ | $S_{3,3}$ | $S_{4,3}$ | $S_{5,3}$ | $S_{6,3}$ | $S_{7,3}$ | $S_{8,3}$ | $S_{9,3}$ | $S_{10,3}$ | $S_{11,3}$ | $\cdots$ | $S_{p,3}$ |
| $S_{1,2}$ | $S_{2,2}$ | $S_{3,2}$ | $S_{4,2}$ | $S_{5,2}$ | $S_{6,2}$ | $S_{7,2}$ | $S_{8,2}$ | $S_{9,2}$ | $S_{10,2}$ | $S_{11,2}$ | $\cdots$ | $S_{p,2}$ |
| $S_{1,1}$ | $S_{2,1}$ | $S_{3,1}$ | $S_{4,1}$ | $S_{5,1}$ | $S_{6,1}$ | $S_{7,1}$ | $S_{8,1}$ | $S_{9,1}$ | $S_{10,1}$ | $S_{11,1}$ | $\cdots$ | $S_{p,1}$ |
| $CH_1$ | $CH_2$ | $CH_3$ | $CH_4$ | $CH_5$ | $CH_6$ | $CH_7$ | $CH_8$ | $CH_9$ | $CH_{10}$ | $CH_{11}$ | $\cdots$ | $CH_p$ |

| $P_{1,1}$ | $P_{1,2}$ | $P_{1,3}$ | $P_{1,4}$ | $P_{1,5}$ | $P_{1,6}$ | $P_{1,7}$ | $P_{1,8}$ | $P_{1,9}$ | $\cdots$ | $P_{1,N}$ |
|---|---|---|---|---|---|---|---|---|---|---|
| $P_{2,1}$ | $P_{2,2}$ | $P_{2,3}$ | $P_{2,4}$ | $P_{2,5}$ | $P_{2,6}$ | $P_{2,7}$ | $P_{2,8}$ | $P_{2,9}$ | $\cdots$ | $P_{2,N}$ |
| $P_{3,1}$ | $P_{3,2}$ | $P_{3,3}$ | $P_{3,4}$ | $P_{3,5}$ | $P_{3,6}$ | $P_{3,7}$ | $P_{3,8}$ | $P_{3,9}$ | $\cdots$ | $P_{3,N}$ |
| $P_{4,1}$ | $P_{4,2}$ | $P_{4,3}$ | $P_{4,4}$ | $P_{4,5}$ | $P_{4,6}$ | $P_{4,7}$ | $P_{4,8}$ | $P_{4,9}$ | $\cdots$ | $P_{4,N}$ |
| $P_{5,1}$ | $P_{5,2}$ | $P_{5,3}$ | $P_{5,4}$ | $P_{5,5}$ | $P_{5,6}$ | $P_{5,7}$ | $P_{5,8}$ | $P_{5,9}$ | $\cdots$ | $P_{5,N}$ |
| $P_{6,1}$ | $P_{6,2}$ | $P_{6,3}$ | $P_{6,4}$ | $P_{6,5}$ | $P_{6,6}$ | $P_{6,7}$ | $P_{6,8}$ | $P_{6,9}$ | $\cdots$ | $P_{6,N}$ |
| $P_{7,1}$ | $P_{7,2}$ | $P_{7,3}$ | $P_{7,4}$ | $P_{7,5}$ | $P_{7,6}$ | $P_{7,7}$ | $P_{7,8}$ | $P_{7,9}$ | $\cdots$ | $P_{7,N}$ |
| $\vdots$ | $\vdots$ | $\vdots$ | $\vdots$ | $\vdots$ | $\vdots$ | $\vdots$ | $\vdots$ | $\vdots$ | $\vdots$ | $\vdots$ |
| $P_{M,1}$ | $P_{M,2}$ | $P_{M,3}$ | $P_{M,4}$ | $P_{M,5}$ | $P_{M,6}$ | $P_{M,7}$ | $P_{M,8}$ | $P_{M,9}$ | $\cdots$ | $P_{M,N}$ |

UI

# FIG. 9

| $S_{1,t}$ | $S_{2,t}$ | $S_{3,t}$ | $S_{4,t}$ | $S_{5,t}$ | $S_{6,t}$ | $S_{7,t}$ | $S_{8,t}$ | $S_{9,t}$ | $S_{10,t}$ | $S_{11,t}$ | $\cdots$ | $S_{p,t}$ |
|---|---|---|---|---|---|---|---|---|---|---|---|---|
| $\vdots$ | $\vdots$ | $\vdots$ | $\vdots$ | $\vdots$ | $\vdots$ | $\vdots$ | $\vdots$ | $\vdots$ | $\vdots$ | $\vdots$ | $\vdots$ | $\vdots$ |
| $S_{1,6}$ | $S_{2,6}$ | $S_{3,6}$ | $S_{4,6}$ | $S_{5,6}$ | $S_{6,6}$ | $S_{7,6}$ | $S_{8,6}$ | $S_{9,6}$ | $S_{10,6}$ | $S_{11,6}$ | $\cdots$ | $S_{p,6}$ |
| $S_{1,5}$ | $S_{2,5}$ | $S_{3,5}$ | $S_{4,5}$ | $S_{5,5}$ | $S_{6,5}$ | $S_{7,5}$ | $S_{8,5}$ | $S_{9,5}$ | $S_{10,5}$ | $S_{11,5}$ | $\cdots$ | $S_{p,5}$ |
| $S_{1,4}$ | $S_{2,4}$ | $S_{3,4}$ | $S_{4,4}$ | $S_{5,4}$ | $S_{6,4}$ | $S_{7,4}$ | $S_{8,4}$ | $S_{9,4}$ | $S_{10,4}$ | $S_{11,4}$ | $\cdots$ | $S_{p,4}$ |
| $S_{1,3}$ | $S_{2,3}$ | $S_{3,3}$ | $S_{4,3}$ | $S_{5,3}$ | $S_{6,3}$ | $S_{7,3}$ | $S_{8,3}$ | $S_{9,3}$ | $S_{10,3}$ | $S_{11,3}$ | $\cdots$ | $S_{p,3}$ |
| $S_{1,2}$ | $S_{2,2}$ | $S_{3,2}$ | $S_{4,2}$ | $S_{5,2}$ | $S_{6,2}$ | $S_{7,2}$ | $S_{8,2}$ | $S_{9,2}$ | $S_{10,2}$ | $S_{11,2}$ | $\cdots$ | $S_{p,2}$ |
| $S_{1,1}$ | $S_{2,1}$ | $S_{3,1}$ | $S_{4,1}$ | $S_{5,1}$ | $S_{6,1}$ | $S_{7,1}$ | $S_{8,1}$ | $S_{9,1}$ | $S_{10,1}$ | $S_{11,1}$ | $\cdots$ | $S_{p,1}$ |
| $CH_1$ | $CH_2$ | $CH_3$ | $CH_4$ | $CH_5$ | $CH_6$ | $CH_7$ | $CH_8$ | $CH_9$ | $CH_{10}$ | $CH_{11}$ | $\cdots$ | $CH_p$ |

| $P_{1,1}$ | $P_{1,2}$ | $P_{1,3}$ | $P_{1,4}$ | $P_{1,5}$ | $P_{1,6}$ | $P_{1,7}$ | $P_{1,8}$ | $P_{1,9}$ | $\cdots$ | $P_{1,N}$ |
|---|---|---|---|---|---|---|---|---|---|---|
| $P_{2,1}$ | $P_{2,2}$ | $P_{2,3}$ | $P_{2,4}$ | $P_{2,5}$ | $P_{2,6}$ | $P_{2,7}$ | $P_{2,8}$ | $P_{2,9}$ | $\cdots$ | $P_{2,N}$ |
| $P_{3,1}$ | $P_{3,2}$ | $P_{3,3}$ | $P_{3,4}$ | $P_{3,5}$ | $P_{3,6}$ | $P_{3,7}$ | $P_{3,8}$ | $P_{3,9}$ | $\cdots$ | $P_{3,N}$ |
| $P_{4,1}$ | $P_{4,2}$ | $P_{4,3}$ | $P_{4,4}$ | $P_{4,5}$ | $P_{4,6}$ | $P_{4,7}$ | $P_{4,8}$ | $P_{4,9}$ | $\cdots$ | $P_{4,N}$ |
| $P_{5,1}$ | $P_{5,2}$ | $P_{5,3}$ | $P_{5,4}$ | $P_{5,5}$ | $P_{5,6}$ | $P_{5,7}$ | $P_{5,8}$ | $P_{5,9}$ | $\cdots$ | $P_{5,N}$ |
| $P_{6,1}$ | $P_{6,2}$ | $P_{6,3}$ | $P_{6,4}$ | $P_{6,5}$ | $P_{6,6}$ | $P_{6,7}$ | $P_{6,8}$ | $P_{6,9}$ | $\cdots$ | $P_{6,N}$ |
| $P_{7,1}$ | $P_{7,2}$ | $P_{7,3}$ | $P_{7,4}$ | $P_{7,5}$ | $P_{7,6}$ | $P_{7,7}$ | $P_{7,8}$ | $P_{7,9}$ | $\cdots$ | $P_{7,N}$ |
| $\vdots$ | $\vdots$ | $\vdots$ | $\vdots$ | $\vdots$ | $\vdots$ | $\vdots$ | $\vdots$ | $\vdots$ | $\vdots$ | $\vdots$ |
| $P_{M,1}$ | $P_{M,2}$ | $P_{M,3}$ | $P_{M,4}$ | $P_{M,5}$ | $P_{M,6}$ | $P_{M,7}$ | $P_{M,8}$ | $P_{M,9}$ | $\cdots$ | $P_{M,N}$ |

$CV_{6,3}$

$UI$

# FIG. 10

# FIG. 11

| $S_{1,t}$ | $S_{2,t}$ | $S_{3,t}$ | $S_{4,t}$ | $S_{5,t}$ | $S_{6,t}$ | $S_{7,t}$ | $S_{8,t}$ | $S_{9,t}$ | $S_{10,t}$ | $S_{11,t}$ | $\cdots$ | $S_{p,t}$ |
|---|---|---|---|---|---|---|---|---|---|---|---|---|
| $\vdots$ | $\vdots$ | $\vdots$ | $\vdots$ | $\vdots$ | $\vdots$ | $\vdots$ | $\vdots$ | $\vdots$ | $\vdots$ | $\vdots$ | $\vdots$ | $\vdots$ |
| $S_{1,6}$ | $S_{2,6}$ | $S_{3,6}$ | $S_{4,6}$ | $S_{5,6}$ | $S_{6,6}$ | $S_{7,6}$ | $S_{8,6}$ | $S_{9,6}$ | $S_{10,6}$ | $S_{11,6}$ | $\cdots$ | $S_{p,6}$ |
| $S_{1,5}$ | $S_{2,5}$ | $S_{3,5}$ | $S_{4,5}$ | $S_{5,5}$ | $S_{6,5}$ | $S_{7,5}$ | $S_{8,5}$ | $S_{9,5}$ | $S_{10,5}$ | $S_{11,5}$ | $\cdots$ | $S_{p,5}$ |
| $S_{1,4}$ | $S_{2,4}$ | $S_{3,4}$ | $S_{4,4}$ | $S_{5,4}$ | $S_{6,4}$ | $S_{7,4}$ | $S_{8,4}$ | $S_{9,4}$ | $S_{10,4}$ | $S_{11,4}$ | $\cdots$ | $S_{p,4}$ |
| $S_{1,3}$ | $S_{2,3}$ | $S_{3,3}$ | $S_{4,3}$ | $S_{5,3}$ | $S_{6,3}$ | $S_{7,3}$ | $S_{8,3}$ | $S_{9,3}$ | $S_{10,3}$ | $S_{11,3}$ | $\cdots$ | $S_{p,3}$ |
| $S_{1,2}$ | $S_{2,2}$ | $S_{3,2}$ | $S_{4,2}$ | $S_{5,2}$ | $S_{6,2}$ | $S_{7,2}$ | $S_{8,2}$ | $S_{9,2}$ | $S_{10,2}$ | $S_{11,2}$ | $\cdots$ | $S_{p,2}$ |
| $S_{1,1}$ | $S_{2,1}$ | $S_{3,1}$ | $S_{4,1}$ | $S_{5,1}$ | $S_{6,1}$ | $S_{7,1}$ | $S_{8,1}$ | $S_{9,1}$ | $S_{10,1}$ | $S_{11,1}$ | $\cdots$ | $S_{p,1}$ |

| $CH_1$ | $CH_2$ | $CH_3$ | $CH_4$ | $CH_5$ | $CH_6$ | $CH_7$ | $CH_8$ | $CH_9$ | $CH_{10}$ | $CH_{11}$ | $\cdots$ | $CH_p$ |
|---|---|---|---|---|---|---|---|---|---|---|---|---|

| $P_{1,1}$ | $P_{1,2}$ | $P_{1,3}$ | $P_{1,4}$ | $P_{1,5}$ | $P_{1,6}$ | $P_{1,7}$ | $P_{1,8}$ | $P_{1,9}$ | $\cdots$ | $P_{1,N}$ |
|---|---|---|---|---|---|---|---|---|---|---|
| $P_{2,1}$ | $P_{2,2}$ | $P_{2,3}$ | $P_{2,4}$ | $P_{2,5}$ | $P_{2,6}$ | $P_{2,7}$ | $P_{2,8}$ | $P_{2,9}$ | $\cdots$ | $P_{2,N}$ |
| $P_{3,1}$ | $P_{3,2}$ | $P_{3,3}$ | $P_{3,4}$ | $P_{3,5}$ | $P_{3,6}$ | $P_{3,7}$ | $P_{3,8}$ | $P_{3,9}$ | $\cdots$ | $P_{3,N}$ |
| $P_{4,1}$ | $P_{4,2}$ | $P_{4,3}$ | $P_{4,4}$ | $P_{4,5}$ | $P_{4,6}$ | $P_{4,7}$ | $P_{4,8}$ | $P_{4,9}$ | $\cdots$ | $P_{4,N}$ |
| $P_{5,1}$ | $P_{5,2}$ | $P_{5,3}$ | $P_{5,4}$ | $P_{5,5}$ | $P_{5,6}$ | $P_{5,7}$ | $P_{5,8}$ | $P_{5,9}$ | $\cdots$ | $P_{5,N}$ |
| $P_{6,1}$ | $P_{6,2}$ | $P_{6,3}$ | $P_{6,4}$ | $P_{6,5}$ | $P_{6,6}$ | $P_{6,7}$ | $P_{6,8}$ | $P_{6,9}$ | $\cdots$ | $P_{6,N}$ |
| $P_{7,1}$ | $P_{7,2}$ | $P_{7,3}$ | $P_{7,4}$ | $P_{7,5}$ | $P_{7,6}$ | $P_{7,7}$ | $P_{7,8}$ | $P_{7,9}$ | $\cdots$ | $P_{7,N}$ |
| $\vdots$ | $\vdots$ | $\vdots$ | $\vdots$ | $\vdots$ | $\vdots$ | $\vdots$ | $\vdots$ | $\vdots$ | $\vdots$ | $\vdots$ |
| $P_{M,1}$ | $P_{M,2}$ | $P_{M,3}$ | $P_{M,4}$ | $P_{M,5}$ | $P_{M,6}$ | $P_{M,7}$ | $P_{M,8}$ | $P_{M,9}$ | $\cdots$ | $P_{M,N}$ |

$CV_{6,4}$

UI

# FIG. 12

# FIG. 13

$P_{3,2}$

$W_1$

$CV_{6,3}$

$W_2$

$P_{4,2}$

$\alpha_1 S_{6,3}$

$\alpha_2 S_{6,3}$

## FIG. 14

# FIG. 15

```
                    ┌─────────┐
                    │  START  │
                    └─────────┘
                         │
                         ▼
        ┌──────────────────────────────────┐
        │  FORMING BRIGHTNESS MODE IMAGE   │────── S1502
        └──────────────────────────────────┘
                         │
                         ▼
        ┌──────────────────────────────────┐
        │    SETTING REGION OF INTEREST    │────── S1504
        └──────────────────────────────────┘
                         │
                         ▼
        ┌──────────────────────────────────┐
        │    FORMING VECTOR INFORMATION    │────── S1506
        └──────────────────────────────────┘
                         │
                         ▼
        ┌──────────────────────────────────┐
        │  FORMING ADDITIONAL INFORMATION  │────── S1508
        └──────────────────────────────────┘
                         │
                         ▼
        ┌──────────────────────────────────┐
        │      SETTING DECISION DATA       │────── S1510
        └──────────────────────────────────┘
                         │
                         ▼
        ┌──────────────────────────────────┐
        │  FORMING VECTOR DOPPLER IMAGE    │────── S1512
        └──────────────────────────────────┘
                         │
                         ▼
                    ┌─────────┐
                    │   END   │
                    └─────────┘
```

FIG. 16

$$\begin{bmatrix} \alpha_{11}x_1 + \alpha_{12}x_2 = y_1 \\ \alpha_{21}x_1 + \alpha_{22}x_2 = y_2 \\ \vdots \\ \alpha_{n1}x_1 + \alpha_{n2}x_2 = y_n \end{bmatrix} \quad \begin{array}{l} \text{If} \\ \text{Tx1 (D1), Rx1(D3)} \\ \text{Tx2 (D2), Rx2(D3)} \end{array}$$

$$\begin{bmatrix} (\alpha_{11} + \alpha_{31})x_1 + (\alpha_{12} + \alpha_{32})x_2 = (y_1 + y_3) \\ (\alpha_{21} + \alpha_{31})x_1 + (\alpha_{22} + \alpha_{32})x_2 = (y_2 + y_3) \\ \vdots \end{bmatrix}$$

$$\vec{y} = A\vec{x} + \vec{n}$$

$$\vec{x}_{opt} = \arg\min_{\vec{x}} (\vec{y} - A\vec{x} - \vec{n})$$

Over-Determined Problem

Pseudo Inverse Method

$$\vec{x}_{opt} = (A^T A)^{-1} A^T \vec{y}$$

EP 2 609 871 A1

**Europäisches Patentamt**
**European Patent Office**
**Office européen des brevets**

## EUROPEAN SEARCH REPORT

Application Number

EP 12 19 9553

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | UDESEN ET AL: "Examples of In Vivo Blood Vector Velocity Estimation", ULTRASOUND IN MEDICINE AND BIOLOGY, NEW YORK, NY, US, vol. 33, no. 4, 27 March 2007 (2007-03-27), pages 541-548, XP022059574, ISSN: 0301-5629, DOI: 10.1016/J.ULTRASMEDBIO.2006.10.014 * abstract * * page 542, left-hand column, paragraph 1 * * page 543, right-hand column, paragraph 1 - page 544, left-hand column, paragraph 2; figures 3-14 * * page 544, right-hand column, paragraph 2 - page 547, right-hand column, paragraph 1 * | 1-4,9-12 | INV. A61B8/06 A61B8/08 A61B6/00 G01S15/89 |
| X | US 2010/069757 A1 (YOSHIKAWA HIDEKI [JP] ET AL) 18 March 2010 (2010-03-18) * paragraphs [0018], [0022], [0024], [0026], [0027], [0030], [0031], [0032], [0034] - [035x]; figures 7,15 * | 1-16 | TECHNICAL FIELDS SEARCHED (IPC) A61B G01S |
| X | US 2009/326379 A1 (DAIGLE RONALD ELVIN [US] ET AL) 31 December 2009 (2009-12-31) * paragraphs [0074], [0077], [0079], [0080], [0089], [0105]; figures 1,3,4-6,10,11 * | 1-16 | |
| X | US 2011/196237 A1 (PELISSIER LAURENT [CA] ET AL) 11 August 2011 (2011-08-11) * paragraphs [0019], [0060], [0066], [0068], [0071] - [0074], [0076], [0080], [0091] - [0093], [0115], [0125] - [0127], [0136]; figures 4,7a-7c,8,9 * * paragraphs [0171] - [0205], [0221] - [0240], [0246], [0247] * | 1-4,9-12 | |

-/--

The present search report has been drawn up for all claims

| Place of search | Date of completion of the search | Examiner |
|---|---|---|
| The Hague | 15 April 2013 | Daoukou, Eleni |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 12 19 9553

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2009/143667 A1 (KOVACS GABOR [AT] ET AL) 4 June 2009 (2009-06-04) * paragraphs [0036], [0058], [0059], [0069]; figures 1,2,3,5 * | 1-16 | |
| A | US 2008/269611 A1 (PEDRIZZETTI GIANNI [IT] ET AL) 30 October 2008 (2008-10-30) * paragraphs [0029], [0030], [0038], [0045], [0047], [0048]; figures 1-4 * | 1-16 | |

TECHNICAL FIELDS
SEARCHED      (IPC)

The present search report has been drawn up for all claims

| Place of search | Date of completion of the search | Examiner |
|---|---|---|
| The Hague | 15 April 2013 | Daoukou, Eleni |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 12 19 9553

This annex lists the patent family members relating to the patent documents cited in the above-mentioned European search report.
The members are as contained in the European Patent Office EDP file on
The European Patent Office is in no way liable for these particulars which are merely given for the purpose of information.

15-04-2013

| Patent document cited in search report | | Publication date | Patent family member(s) | | Publication date |
|---|---|---|---|---|---|
| US 2010069757 | A1 | 18-03-2010 | CN | 101636113 A | 27-01-2010 |
| | | | EP | 2151194 A1 | 10-02-2010 |
| | | | JP | 4787358 B2 | 05-10-2011 |
| | | | US | 2010069757 A1 | 18-03-2010 |
| | | | WO | 2008136201 A1 | 13-11-2008 |
| US 2009326379 | A1 | 31-12-2009 | CA | 2728998 A1 | 30-12-2009 |
| | | | CN | 102123668 A | 13-07-2011 |
| | | | EP | 2303131 A1 | 06-04-2011 |
| | | | JP | 2011526181 A | 06-10-2011 |
| | | | KR | 20110027806 A | 16-03-2011 |
| | | | US | 2009326379 A1 | 31-12-2009 |
| | | | WO | 2009158399 A1 | 30-12-2009 |
| US 2011196237 | A1 | 11-08-2011 | NONE | | |
| US 2009143667 | A1 | 04-06-2009 | CN | 101444419 A | 03-06-2009 |
| | | | DE | 102007057553 A1 | 10-06-2009 |
| | | | US | 2009143667 A1 | 04-06-2009 |
| US 2008269611 | A1 | 30-10-2008 | CN | 101297762 A | 05-11-2008 |
| | | | EP | 1985238 A2 | 29-10-2008 |
| | | | US | 2008269611 A1 | 30-10-2008 |

EPO FORM P0459

For more details about this annex : see Official Journal of the European Patent Office, No. 12/82

**EP 2 609 871 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020110144432